# EUROPEAN PATENT SPECIFICATION

(11) **EP 3 119 792 B1**
(45) Date of publication and mention of the grant of the patent: **09.01.2019**
(21) Application number: 16731010.1
(22) Date of filing: 10.06.2016
(51) Int. Cl.: C07H 1/08, C08H 8/00, C13B 20/00, C13K 1/02, C13K 1/04

(54) **PROCESS FOR ISOLATING FERMENTABLE SUGARS FROM THE ACID HYDROLYZATE OF A MATERIAL CONTAINING CELLULOSE, HEMICELLULOSE AND LIGNIN**
VERFAHREN ZUR ISOLIERUNG VON FERMENTIERBAREN ZUCKERN AUS DEM SÄUREHYDROLYSAT EINES CELLULOSEHALTIGEN MATERIALS, HEMICELLULOSE UND LIGNIN
PROCÉDÉ POUR ISOLER DES SUCRES FERMENTESCIBLES À PARTIR DE L'HYDROLYSAT ACIDE D'UNE MATIÈRE CONTENANT DE LA CELLULOSE, DE L'HÉMICELLULOSE ET DE LA LIGNINE

(30) Priority: 11.06.2015 IN 2925CH2015
(43) Date of publication of application: 25.01.2017
(73) Proprietor: Petiva Private Limited, 500033 Hyderabad (IN)
(72) Inventor: PANDEY, Banibrata, Hyderabad Telangana 500033 (IN); GIRI, Binoy, Kumar, Hyderabad Telangana 500033 (IN); SUDHAKARAN, D Samuel, Hyderabad Telangana 500033 (IN)
(74) Representative: Wynne-Jones IP Limited
(86) International application number: PCT/IB2016/053431
(87) International publication number: WO 2016/199083

(56) References cited:
- WO-A1-2014/105610
- WO-A1-2014/105610
- WO-A2-2010/146331
- WO-A2-2010/146331
- US-A1- 2008 102 502
- US-A1- 2008 102 502

## Description

This application claims the benefit of Indian provisional application number, 2925/CHE/2015, filed on June 11, 2015.

### FIELD OF THE INVENTION

The present invention relates to a process for isolating fermentable sugars from the acid hydrolyzate of a material containing cellulose, hemicellulose and lignin, particularly, to a process for producing fermentable sugars without contamination of the acid used in the hydrolysis.

### BACKGROUND OF THE INVENTION

Most of the fuel and chemicals produced today are derived from hydrocarbon sources. Sustainability of such technologies is the major disadvantage. The raw material availability is limited and the ever increasing demand for fuels and chemicals has resulted in exploration of different avenues for possible solution. Lignocellulosic biomass, the most abundant renewable raw material on earth, such as agriculture residues, forestry waste, wood etc., is potentially the ideal feedstock for the production of fuels, chemicals and value added chemicals. Lignocellulosic biomass comprises the carbohydrate polymers cellulose and hemicellulose and an aromatic polymer lignin. Intensive research has been carried out over the years to convert the carbohydrate polymers of the lignocellulosic biomass, by mechanical, thermo-chemical or enzymatic means, into sugars which can be fermented or chemically converted to fuels and chemicals. However, these attempts have not resulted in an economically viable process.

Concentrated acid processes have been successful in yielding high amount of sugar but process control requires precision to overcome the following difficulties:
- Formation of sugar degradation products;
- Separation of acid from the sugar produced using membranes or ion exchange resins or solvent extraction of acid or other elaborate mechanisms;
- Recovery of acid used;
- Generation of huge amount of CaSO4 or such salts during neutralization which is difficult to dispose;
- Specialized reactor for corrosive nature of acid;
- Diluted product streams which require further concentration steps; and
- Expensive and not economically viable.

US patent 4608245 discloses a process for recovering concentrated sulfuric acid from the hydrolyzate obtained after acid hydrolysis of cellulose containing biomass. The method involves extracting the acid from the hydrolyzate with one or more of the C₄-C₇ alcohols.

US patent 4645658 discloses a method for recovering hydrochloric acid from the hydrolyzate obtained after acid hydrolysis of cellulose containing biomass. The method involves extracting the acid with a solvent, of which a major portion is acetophenone, to separate into a hydrochloric acid enriched phase and a hydrochloric acid depleted phase and then separating and recovering the hydrochloric acid from the hydrochloric acid enriched phase.

US patent 5820687 discloses a process for producing sugar using concentrated sulfuric acid hydrolysis of biomass and separating the sugars from the acid. The sugars in the hydrolyzate were separated from the acid with the help of a strong acid separation unit. The resin separation unit is preferably a cross-linked polystyrene cation exchange resin bed, wherein the resin is cross linked with divinylbenzene and treated with sulfuric acid to produce a strong acid resin. The hydrolyzate is added to the resin bed, and the sugars are adsorbed onto the resin. The resin is then purged with a gas which pushes the acid out of the resin before the washing step with water, which removes the sugars from the resin.

US patent 7442359 B2 discloses a process for recovery of sulfuric acid from a mixture of sulfuric acid and carbohydrates. The mixture comprising sulfuric acid and carbohydrates is contacted with an anionic selective membrane, which produces a sulfuric acid rich filtrate stream and a stream depleted in sulfuric acid.

US patent 6419828 B1 discloses a method for separating acid and sugars obtained from liquids resulting from the acid hydrolysis of biomass. In this method the liquids were added to a separation using comprising a bed of anionic exchange or exclusion chromatography material whereby the acid was adsorbed onto the chromatographic material and produces a series of fraction comprising sugar solution and later a series of fractions comprising acid solutions.

WO2010146331 discloses a process for producing alcohol from a cellulosic material, said process comprising: hydrolyzing said cellulosic material with an aqueous acid to produce a hydrolysate; extracting acid and water from said hydrolysate with a water-miscible organic extraction solvent to yield (a) a first aqueous acidic solution containing said extraction solvent and (b) a residue containing sugars; subjecting said residue to an oligosaccharide cleavage reaction to yield an aqueous solution of fermentable sugars; fermenting said fermentable sugars and distilling alcohol from the resulting fermented mixture; contacting said first aqueous acidic solution with a water-immiscible liquid lipophilic solvent to yield a second aqueous acid solution and a solvent mixture of said extraction solvent and said liquid solvent; separating said solvent mixture to yield extraction solvent for recycling; and separating from said second aqueous acid solution an aqueous acid for recycling.

The above prior art references disclose different methods for the isolation of sugars from the acid hydrolyzate of a material containing cellulose and hemicellulose. The recovery of acid is not commercially attractive as the process requires various means and methods to recover and concentrate for further reuse. Hence there is a need for commercially viable process to meet the industry requirements.

### SUMMARY OF THE INVENTION

Accordingly, the present invention provides a process for isolating fermentable sugars from the acid hydrolyzate of a material containing cellulose, hemicellulose and lignin, which comprises:
(a) neutralizing the hydrolyzate with an alkali and filtering the resultant mixture to recover lignin as a solid;
(b) drying the filtrate of step (a) to obtain a residue comprising fermentable sugars and salts;
(c) extracting fermentable sugars from the residue with C₁-C₆ alcohol or their mixtures; and
(d) evaporating the alcohol to yield fermentable sugars.

In one aspect, the present invention relates to a process for producing fermentable sugars without contamination of the acid used in the hydrolysis.

In another aspect, the present invention provides simple, economical and commercially viable process.

### BRIEF DESCRIPTION OF THE ACCOMPANYING DRAWINGS

Figure 1: Mass balance of the example 1
Figure 2: Recovery of fermentable sugars after the extraction process.
Figure 3: HPLC chromatogram of hydrolyzate before neutralization.

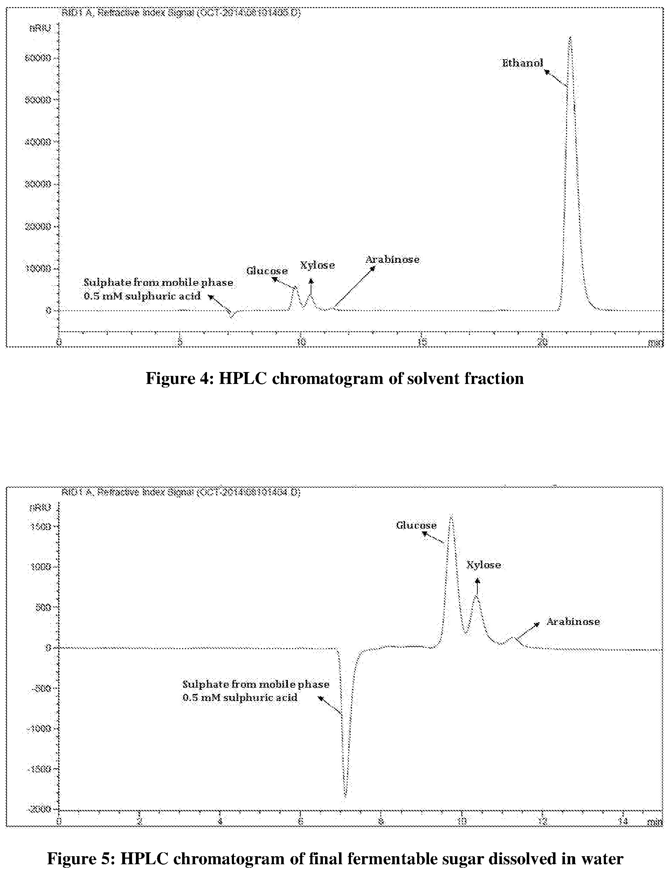

Figure 4: HPLC chromatogram of solvent fraction.
Figure 5: HPLC chromatogram of final fermentable sugar dissolved in water.

### DETAILED DESCRIPTION OF THE INVENTION

In an embodiment, the present invention provides a process for isolating fermentable sugars from the acid hydrolyzate of a material containing cellulose, hemicellulose and lignin, which comprises:
(a) neutralizing the hydrolyzate with an alkali and filtering the resultant mixture to recover lignin as a solid;
(b) drying the filtrate of step (a) to obtain a residue comprising fermentable sugars and salts;
(c) extracting fermentable sugars from the residue with C₁-C₆ alcohol or their mixtures; and
(d) evaporating the alcohol to yield fermentable sugars.

### Step (a)

In an exemplary embodiment, the neutralization of the hydrolyzate is carried out with an alkali. The alkali is selected from a group comprising alkaline hydroxides, alkaline salts, amines and combinations thereof. In one embodiment the alkaline hydroxide is selected from a group comprising ammonium hydroxide, sodium hydroxide, potassium hydroxide, calcium hydroxide, barium hydroxide and combinations thereof. In another embodiment, the alkaline salt is selected from a group comprising sodium borate, sodium carbonate, sodium phosphate, potassium borate, potassium carbonate, potassium phosphate, sodium acetate, sodium citrate and combinations thereof. In yet another embodiment, the amine is selected from a group comprising diethylamine, triethylamine, butylamine, ethylenediamine, trietha-anolamine, propylamine, dipropylamine, diethanol-amine, monoethanolamine, isobutylamine, diisopropylamine, tert-butylamine, dibutylamine, diiso-butylamine, tributylamine, pentylamine, dipentyl-amine and combinations thereof. In a preferred embodiment, the alkali is ammonium hydroxide, sodium hydroxide, potassium hydroxide, calcium hydroxide or barium hydroxide, more preferably ammonium hydroxide. In another embodiment, the neutralization is carried out at ambient temperature or below. In yet another embodiment, the temperature is of about 0 °C to 30 °C or 0 °C to 20 °C or 0 °C to 10 °C. In neutralization step, the alkali was slowly added to the hydrolyzate. Further, the lignin was recovered from the resultant mixture through a method known to those of skilled in the art (e.g. filtration) to collect the filtrate.

### Step (b)

In an exemplary embodiment, the filtrate was dried using a method known to those of skilled in the art (e.g. evaporation under vacuum) to get a residue comprising fermentable sugars and salts.

### Step (c)

In an exemplary embodiment, the residue obtained in step (b) was extracted with a solvent C₁-C₆ alcohol or mixtures thereof. In one embodiment, the solvent is selected from methanol, ethanol, propanol, iso-propanol, butanol, t-butanol and amyl alcohol. In a particular embodiment, the solvent is ethanol. In a further particular embodiment, the alcohol concentration is more than 70% v/v in water.

### Step (d)

In an exemplary embodiment, the solvent extract obtained in step (d) was evaporated using a method known to those of skilled in the art (e.g. drying under vacuum) to yield fermentable sugars. The evaporated solvent can be reused.

In one embodiment, the fermentable sugar is selected from a group comprising glucose, xylose and arabinose.

The skilled practitioner will recognize several parameters of the foregoing processes that may be varied advantageously in order to obtain a desirable outcome. These parameters include, for example, the methods and means of purification of reaction components and solvents; the order of addition of said reaction components and solvents to the reaction mixture; the duration of reaction of said reaction components and solvents; and the temperature and rate of stirring, mixing or agitation of the reaction components and solvents during said reaction.

It was found that the process embodied by the steps (a)-(d) fulfils one or more of the following criteria: simpler, higher yielding and more economical when compared to the known processes for isolating the sugars from the acid hydrolyzate of a material containing cellulose and hemicellulose. Further, the process as described herein is considered scalable, making it suitable for commercial production.

Unless defined otherwise, all technical and scientific terms used herein have the same meaning as is commonly understood by one of skilled in the art to which the subject matter herein belongs. As used in the specification, unless specified to the contrary, the following terms have the meaning indicated in order to facilitate the understanding of the present invention.

The singular forms "a", "an" and "the" encompass plural references unless the context clearly indicates otherwise.

As used herein, the term "comprise" or "comprises" or "comprising" is generally used in the sense of include, that is to say permitting the presence of one or more features or components.

As used herein, the term "hydrolyzate" refers to a product of hydrolysis.

The abbreviations used in the entire specification may be summarized herein below with their particular meaning.

mL (millilitre); w/v (weight/volume); w/w (weight/weight); °C (degree Celsius); g (gram); v/v (volume/volume) and HPLC (High Performance Liquid Chromatography).

### Examples

The following example(s) illustrate the invention without limiting the scope thereof. It is understood that the invention is not limited to the embodiments set forth herein, but embraces all such forms thereof as come within the scope of the disclosure.

### General procedure:

The hydrolyzate obtained after the hydrolysis reaction is neutralized with an alkali, preferably with ammonia. After the neutralization process the solution is filtered to recover the lignin in solid form. The filtrate obtained contains fermentable sugars, ammonium sulfate or calcium sulfate or other salts depending on the alkali used for the neutralization process. The filtrate is dried using any means known in prior art such as vacuum evaporation, spray drying etc., to obtain a residue, which comprises mainly fermentable sugars resulting from the hydrolysis of cellulose and hemicellulose and salt obtained from the neutralization process. The residue is then extracted once or multiple times with a solvent, the solvent used is preferably from C₁-C₆ alcohols or their mixtures. The extraction results in an extract containing the fermentable sugars and a residue comprising mainly the salt formed during neutralization and negligible or no sugars. The extraction is carried out till there is no sugar observed in the extract phase. The extract phase was evaporated under reduced pressure to yield fermentable sugars. The sugar solution produced can be decolorized using an activated charcoal bed.

### Example 1

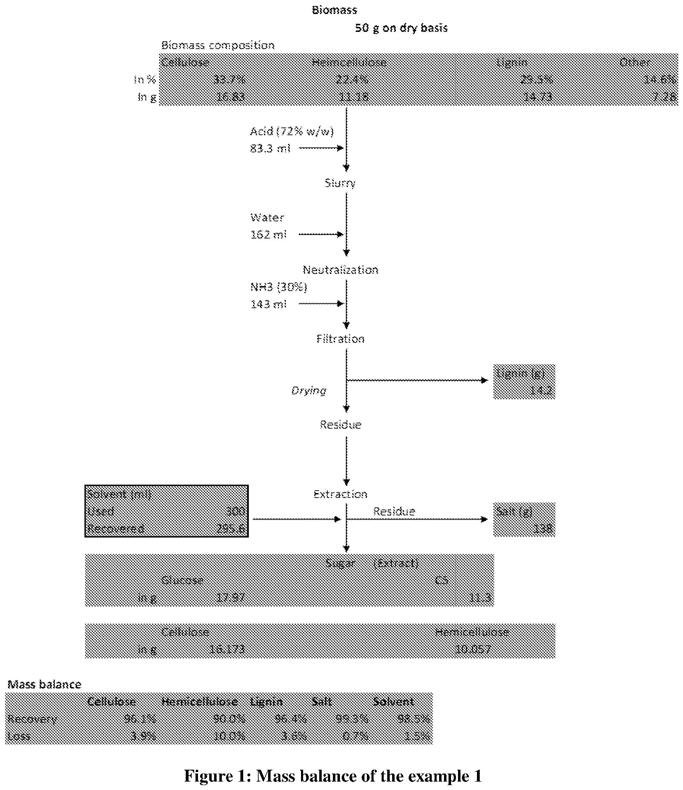

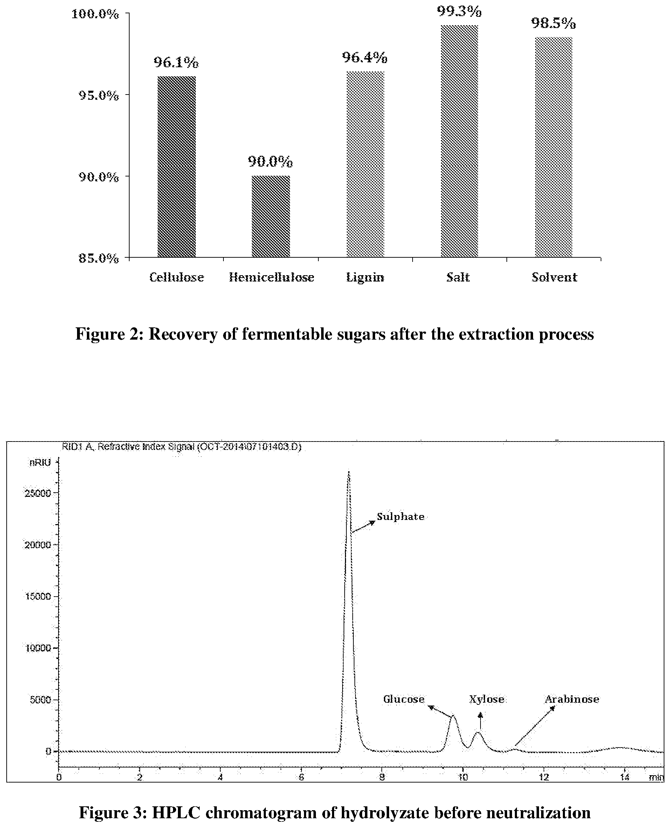

About 50 g of Lignocellulosic biomass (≈ 10% moisture content) was taken. To this biomass 83.3 ml of 72% (w/w) sulfuric acid was added such that the final solid concentration was 60% (w/v) and incubated at 50 °C for 2 h. After the said incubation period the mixture was diluted to a final acid concentration of about 4-5% (w/w) by adding 162 mL of water, and then the mixture was incubated at 90 °C for a period of 80 minutes. The mixture was then cooled to room temperature and neutralized with an alkali (preferably with ammonia, 143 mL of 25% ammonia solution). The neutralized solution was filtered to recover the lignin as a solid. The filtrate containing fermentable sugars and ammonium sulfate (if ammonia is used) was dried under vacuum (at 60 °C). The residue obtained was extracted repeatedly with ethanol, to recover the fermentable sugars. The extraction was continued till there is no more sugar detected in the solvent fraction. The remaining residue was dried at room temperature to obtain almost pure ammonium sulfate. The solvent fraction was evaporated under vacuum to get fermentable sugars. A mass balance of the experiment was given in figure 1 and recovery of sugars is given in figure 2.

### ADVANTAGES OF THE PRESENT PROCESS

1. The present process does not require expensive enzymes and additives.
2. The sugars isolated by this process do not have an acidic impurity.
3. Purity of sugar obtained by this process is high (> 99%).
4. No residual lignin is present in the sugar solution
5. The cost of acid and ammonia used in the process is nullified by the production of byproducts (ammonium sulfate + lignin).
6. The byproduct can be used, alone or mixed with lignin produced in the process, as a fertilizer.
7. About 95-98% of the solvent used for extracting the sugars can be recovered.

## Claims

1. A process for isolating fermentable sugars from the hydrolyzate obtained from the acid hydrolysis of a material containing cellulose, hemicellulose and lignin; wherein the process comprises:
(a) neutralizing the hydrolyzate with an alkali and filtering the resultant mixture to recover lignin as a solid;
(b) drying the filtrate of step (a) to obtain a residue comprising fermentable sugars and salts;
(c) extracting fermentable sugars from the residue with C₁-C₆ alcohol or their mixtures; and
(d) evaporating the alcohol to yield fermentable sugars.

2. The process as claimed in claim 1, wherein the alkali is selected from a group comprising alkaline hydroxides, alkaline salts, amines and combinations thereof.

3. The process as claimed in claim 1, wherein the alkali is alkaline hydroxide, preferably ammonium hydroxide.

4. The process as claimed in claim 1, wherein the alcohol is ethanol.

5. The process as claimed in claim 1, wherein the alcohol concentration is more than 70% v/v in water.

6. The process as claimed in claim 1, wherein the neutralization is carried out at ambient temperature or below.

7. The process as claimed in claim 1, wherein the neutralization is carried out at a temperature of about 0 °C to 10 °C.

8. The process as claimed in claim 1, wherein the sugar is selected from a group comprising glucose, xylose and arabinose.

## Patentansprüche

1. Verfahren zum Isolieren von fermentierbaren Zuckern aus dem Hydrolysat, das durch eine Säurehydrolyse eines Materials erhalten wird, das Cellulose, Hemicellulose und Lignin enthält, wobei das Verfahren umfasst:
(a) Neutralisieren des Hydrolysats mit einem Alkali und Filtrieren des resultierenden Gemischs zum Isolieren von Lignin als Feststoff;
(b) Trocknen des Filtrats von Schritt (a) zum Erhalten eines Rückstands, der fermentierbare Zucker und Salze umfasst;
(c) Extrahieren von fermentierbaren Zuckern aus dem Rückstand mit einem C₁-C₆-Alkohol oder deren Gemischen; und
(d) Verdampfen des Alkohols zum Erhalten von fermentierbaren Zuckern.

2. Verfahren nach Anspruch 1, bei dem das Alkali aus einer Gruppe, umfassend Alkalihydroxide, Alkalisalze, Amine und Kombinationen davon, ausgewählt ist.

3. Verfahren nach Anspruch 1, bei dem das Alkali ein Alkalihydroxid, vorzugsweise Ammoniumhydroxid, ist.

4. Verfahren nach Anspruch 1, bei dem der Alkohol Ethanol ist.

5. Verfahren nach Anspruch 1, bei dem die Alkoholkonzentration mehr als 70 % V/V in Wasser beträgt.

6. Verfahren nach Anspruch 1, bei dem die Neutralisation bei Umgebungstemperatur oder darunter durchgeführt wird.

7. Verfahren nach Anspruch 1, bei dem die Neutralisation bei einer Temperatur von etwa 0 °C bis 10 °C durchgeführt wird.

8. Verfahren nach Anspruch 1, bei dem der Zucker aus einer Gruppe, umfassend Glukose, Xylose und Arabinose, ausgewählt ist.

## Revendications

1. Procédé pour isoler des sucres fermentescibles à partir de l'hydrolysat obtenu par l'hydrolyse acide d'une matière contenant de la cellulose, de l'hémicellulose et de la lignine, le procédé comprenant :
(a) neutraliser l'hydrolysat par un alcali et filtrer le mélange résultant pour récupérer la lignine en tant que solide ;
(b) sécher le filtrat de l'étape (a) pour obtenir un résidu comprenant des sucres fermentescibles et des sels ;
(c) extraire les sucres fermentescibles à partir du résidu avec un alcool en C₁-C₆ ou leurs mélanges ; et
(d) faire évaporer l'alcool pour obtenir les sucres fermentescibles.

2. Procédé selon la revendication 1, dans lequel l'alcali est choisi dans un groupe comprenant les hydroxydes alcalins, les sels alcalins, les amines et les combinaisons de ceux-ci.

3. Procédé selon la revendication 1, dans lequel l'alcali est un hydroxyde alcalin, de préférence l'hydroxyde d'ammonium.

4. Procédé selon la revendication 1, dans lequel l'alcool est l'éthanol.

5. Procédé selon la revendication 1, dans lequel la concentration en alcool est supérieure à 70 % v/v dans l'eau.

6. Procédé selon la revendication 1, dans lequel la neutralisation est effectuée à la température ambiante ou au-dessous.

7. Procédé selon la revendication 1, dans lequel la neutralisation est effectuée à une température d'environ 0°C à 10°C.

8. Procédé selon la revendication 1, dans lequel le sucre est choisi dans un groupe comprenant le glucose, le xylose et l'arabinose.
